# EUROPEAN PATENT SPECIFICATION

(11) **EP 1 922 005 B1**
(45) Date of publication and mention of the grant of the patent: **21.12.2011**
(21) Application number: 06795624.3
(22) Date of filing: 09.08.2006
(51) Int. Cl.: A61B 18/14

(54) **SYSTEM FOR ELECTROPHYSIOLOGY REGAINING SUPPORT TO CONTINUE LINE AND RING ABLATIONS**
SYSTEM ZUR ELEKTROPHYSIOLOGISCHEN WIEDERGEWINNUNG EINER STÜTZE ZUR FORTFÜHRUNG VON LINIEN- UND RING-ABLEITUNGEN
SYSTEME POUR SUPPORT DE RETABLISSEMENT DE L'ELECTROPHYSIOLOGIE AFIN DE CONTINUER DES ABLATIONS LINEAIRES ET D'ANNEAU

(30) Priority: 25.08.2005 US 711320 P
(43) Date of publication of application: 21.05.2008
(73) Proprietor: Koninklijke Philips Electronics N.V., 5621 BA Eindhoven (NL); Philips Intellectual Property & Standards GmbH, 20099 Hamburg (DE)
(72) Inventor: BREDNO, Joerg, NL-5621 BA Eindhoven (NL); ECK, Kai, NL-5621 BA Eindhoven (NL)
(74) Representative: Uittenbogaard, Frank
(86) International application number: PCT/IB2006/052756
(87) International publication number: WO 2007/023407

(56) References cited:
- EP-A2- 1 415 608
- US-A- 5 588 432
- US-A1- 2005 038 333

## Description

The present disclosure relates generally to a system for repositioning an ablation catheter to points on the cardiac tissue where contact with the catheter was lost, in order to continue line or ring ablations in the treatment of tachycardia.

Tachycardia can be caused by abnormal conduction of the electric pulse, where the pulse doesn't follow its physiological pathway but creates feedback loops, e.g. from one of the ventricles back to the atrium (reentry tachycardia) or by non-physiologic circular conduction pathways in one of the ventricles e.g. around scar tissue or in one of the atria, resulting in a high heart rate. A ring or line ablation is required to block reentry tachycardia or abnormal conduction pathways, and there must be no gaps in the ablation path.

Electrophyisologic (EP) diagnosis and treatment of cardiac arrhythmia receives more and more clinical attention. Tachycardia (irregular increases of the pulse rate with irregular heart beat configuration) requires treatment because it has been identified as a major source for small blood coagulations that induce a high risk of stroke or cardiac infarction. Sources of tachycardia can be either ectotopic (local diseased heart tissue that creates false impulses) or due to reentry conduction where the electric pulse does not follow its physiologic pathways but creates parasitic feedback loops that result in a pathologically high heart rate.

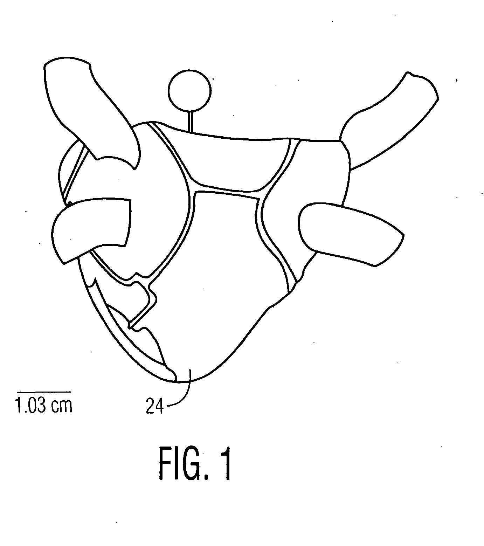

Cardiac mapping is used to locate aberrant electrical pathways and currents within the heart, as well as to diagnose mechanical and other aspects of cardiac activity. Various methods and devices have been described for mapping the heart. Radiofrequency (RF) ablation is used to treat cardiac arrhythmia by ablating and killing cardiac tissue in order to create non-conducting lesions that disrupt the abnormal electrical pathway causing the arrhythmia. In RF ablation, heat is induced at the tip of an ablation catheter to create lesions in the myocardium. Such ablated scar tissue can no longer create or transport electric impulses. Local ablation destroys irregular local sources, whereas a ring or line ablation is required to block reentry tachycardia. Figure 1 depicts what is commonly referred to as a cartoon image of localizer information relating to an ablation procedure in the left atrium of a patient's heart. The line traversing and forming rings about the heart tissue indicate positions where an ablation-induced block was intended by the physician.

Line and ring ablations are extremely time-intense, lasting hours because any gap in the disabled tissue can cause a continued reentry tachycardia. It is desired that the intervention allow for a fast revisit of candidate positions where the ablation catheter was not in sufficient contact with the heart tissue when ablation was intended by the interventionalist.

It is to be noted that EP 1415608 A2 discloses a real-time cardiac ablation mapping system comprising a catheter and a display monitor. Herein, the catheter may comprise a position sensor, a tip electrode and one or more temperature sensors. During a cardiac ablation procedure, the catheter is inserted into the cardiac chamber, and is used, during a plurality of cardiac cycles, to acquire and record position information and information about a power dose applied by the tip electrode during ablation, including power information and temperature information generated by the temperature sensors. Using the position and power dose information, a three-dimensional reconstruction of the ablation lesion is generated and displayed in real time on the monitor.

It is furthermore to be noted that US 2005/0038333 A1 discloses a catheter apparatus for use in the treatment of heart arrhythmia having an ablation catheter. The ablation catheter includes an electrode section at its distal end. This electrode section has a number of electrodes, each electrode able to ablate the heart tissue with which it is brought into contact. The electrodes may be employed with both a temperature recording site and an electrogram recording site.

It is to be noted that US-A-588432 discloses an ultrasound and electrophysiology catheter connected to an ultrasound imaging system that receives signals from an ultrasound transducer and transmits image data to a display system. By observing in real time on the display system, the region of the heart near the ultrasound and electrophysiology catheter, a physician can determine the position of the catheter relative to the cardiac tissue and can also reposition the catheter at the same location at a later time.

The present disclosure provides an apparatus for ablating tissue in a heart (24) of a subject (25) during an ablation procedure. The apparatus includes: an ablation catheter tip (48) contacting tissue of the heart (24) at a plurality of sites designated for ablation; a sensor means for sensing at each respective site electrical current required to maintain the tip (48) at a target temperature; a storage means for storing any available data defining a current position of the ablation catheter tip (48) relative to the heart (24) at a moment of sensing the required electrical current above a threshold current value for later re-visit; and a display means (60) for displaying a map of a region of interest of the heart (24), wherein indications of the sites corresponding to when the required electrical current is above the threshold current value indicative of a gap in an ablation line or ring are designated on the display means.

The present disclosure also provides a computer software product (100) for ablating tissue in a heart (24) of a subject (25) during an ablation procedure. The product includes a computer-readable medium, in which program instructions are stored, which instructions, when read by a computer, cause the computer (50) to: sense electrical current required to maintain an ablation catheter tip (48) at a target temperature at a plurality of sites designated for ablation during an ablation procedure; store any available data defining a current position of the ablation catheter tip (48) relative to the heart (24) at a moment of sensing the required electrical current above a threshold current value for later re-visit; display a map (60) of a region of interest of the heart (24); and designate, on the map display (60), indications of the sites corresponding to when the required electrical current is above the threshold current value indicative of a gap in an ablation line or ring.

Additional features, functions and advantages associated with the disclosed system will be apparent from the detailed description which follows, particularly when reviewed in conjunction with the figures appended hereto.

To assist those of ordinary skill in the art in making and using the disclosed system, reference is made to the appended figures, wherein:
FIGURE 1 depicts an intended ablation path indicated as dots on a so-called cartoon image of the left atrium illustrating where an ablation-induced block is intended by the physician;
FIGURE 2 is a schematic, pictorial illustration of a system for real-time mapping of cardiac ablation treatment in the heart, in accordance with an exemplary embodiment of the present disclosure;
FIGURE 3 is a schematic, pictorial illustration of a distal portion of a catheter used in the system of FIG. 2, in accordance with an exemplary embodiment of the present disclosure;
FIGURE 4 is a flow chart that schematically illustrates a method for indicating gaps formed during line or ring ablation in a cardiac chamber for immediate or later re-visit, in accordance with an exemplary embodiment of the present disclosure;
FIGURE 5 illustrates a fluoroscopy x-ray image that is automatically acquired and displayed or stored upon detection of a gap in the line or ring ablation procedure indicative of a catheter tip losing contact with the heart tissue, in accordance with an exemplary embodiment of the present disclosure.

As set forth herein, the present disclosure advantageously facilitates detection of the loss of contact between the catheter tip and heart tissue using an automated navigation support to revisit those parts of the ablation line or ring where gaps are possible. The present disclosure may be advantageously employed in cardio applications including automated acquisition and storage of position information at the moment where ablation contact to the heart tissue is lost serving to massively reduce the amount of time that is spent in trial and error corrections of incomplete ring and line ablations to treat reentry tachycardia.

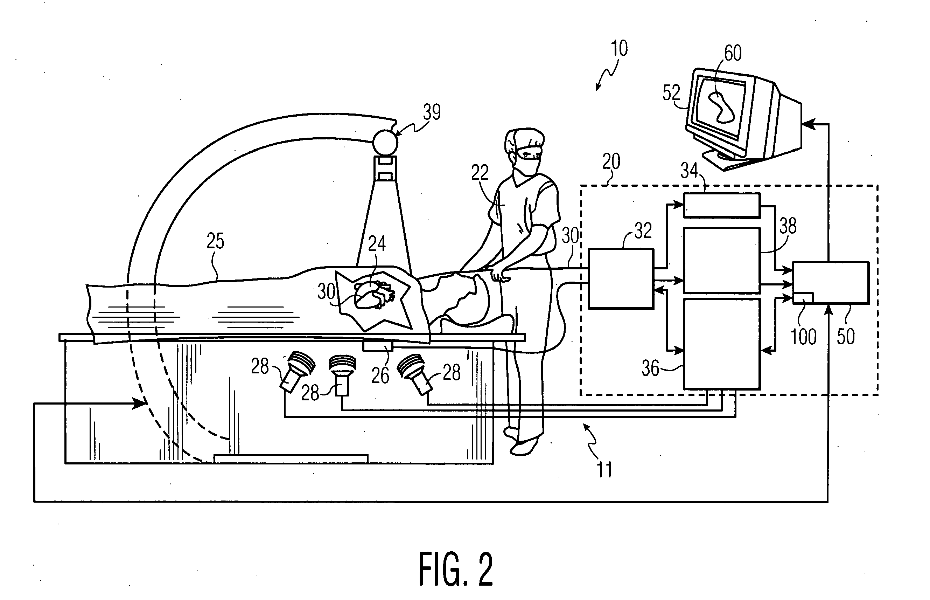

Figure 2 is a schematic, pictorial illustration of a mapping system 10, for real-time mapping of cardiac ablation treatment in a heart 24 of a subject 25, in accordance with an exemplary embodiment of the present disclosure. System 10 comprises an elongated mapping probe, preferably a catheter 30, which is inserted by a user 22 through a vein or artery of the subject into a chamber of the heart, which can be the right or left ventricle or atrium.

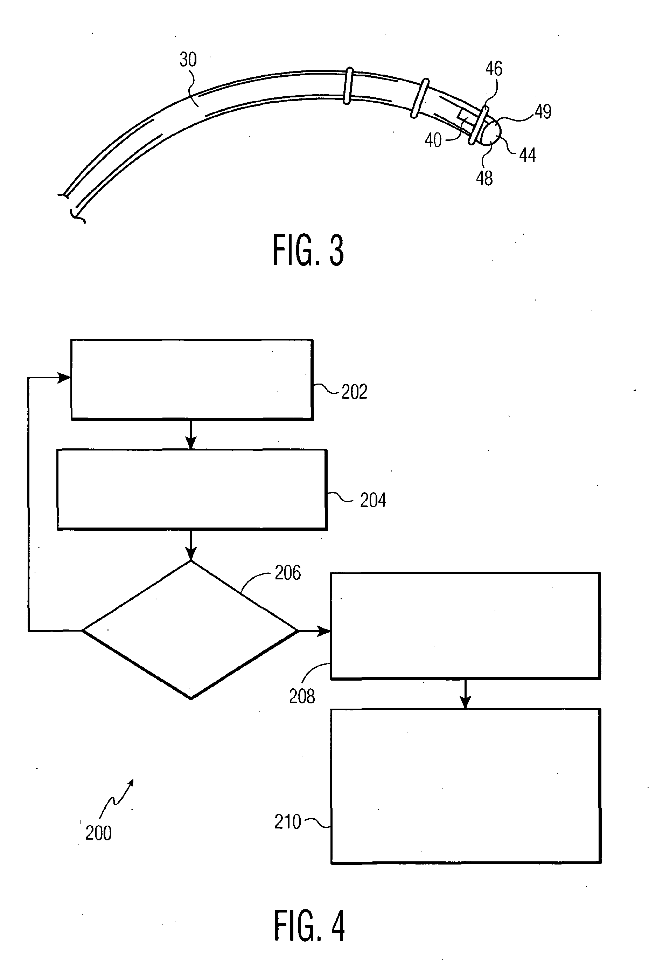

Figure 3 is a schematic, pictorial illustration showing a distal portion of catheter 30, which is inserted into heart 24. Catheter 30 preferably comprises at least one position sensor 40, a tip electrode 48, and one or more temperature sensors 49, all of which are preferably fixed at or near a distal tip 44 of the catheter. Temperature sensors 49 may comprise, for example, thermocouples and/or thermistors. Position sensor 40 generates or receives signals used to determine the position and orientation of catheter 40 within the chamber of the heart. Tip electrode 48 is preferably configured to apply electrical signals to heart 24 for ablating cardiac tissue, and is preferably further configured for diagnostic purposes such as cardiac mapping. Alternatively, separate electrodes are provided for diagnostic purposes and for ablating cardiac tissue. There is preferably a fixed positional and orientational relationship of position sensor 40, distal tip 44 and tip electrode 48. Optionally, catheter 30 further comprises at least one additional position sensor (not shown) and radio-opaque markers to identify individual catheters and to determine their location and orientation on x-ray projection images.

Reference is again made to Figure 2. In a preferred embodiment of the present invention, mapping system 10 comprises a display monitor 52, an imaging system 39 and a console 20, which preferably comprises a location system control unit 36, an ablation power generator 38, a junction box 32, an electrocardiogram (ECG) recording and/or monitoring system 34 and a computer 50, which preferably comprises appropriate signal processing circuits that are typically contained inside a housing of the computer. Computer 50 is preferably programmed in software and/or hardware to carry out the functions described herein. This software may be downloaded to the computer in electronic form, over a network, for example, or it may alternatively be provided on tangible media, such as magnetic or optical media or other non-volatile memory. In some embodiments, computer 50 comprises a general-purpose computer.

Junction box 32 preferably routes (a) conducting wires and temperature sensor signals from catheter 30 to ablation power generator 38, (b) location sensor information from sensor 40 of catheter 30 to location system control unit 36, and (c) the diagnostic electrode signals generated by tip electrode 48 to ECG monitor 34. Alternatively or additionally, junction box 32 routes one or more of these signals directly to computer 50. ECG monitor 34 is preferably also coupled to receive signals from one or more body surface electrodes, so as to provide an ECG synchronization signal to computer 50.

The imaging system 39 is further operably connected to computer 50 for control and receipt of images from the imaging system 39. In an exemplary embodiment, imaging system is a fluoroscopy x-ray system. However, other imaging modalities are contemplated including, but not limited to, MRI, echocardiography, CT, or any other modality suitable to provide an instantaneous image that captures the current position of the catheter together with heart tissue.

A location system 11 preferably comprises a set of external radiators 28, position sensor 40 of catheter 30 and any additional position sensors, and location system control unit 36. External radiators 28 are preferably adapted to be located at respective positions external to subject 25 and to generate fields, such as electromagnetic fields, towards position sensor 40, which is adapted to detect the fields and facilitate a calculation of its position coordinates by location system control unit 36 responsive to the fields. Alternatively, position sensor 40 generates fields, which are detected by external radiators 28. For some applications, a reference position sensor, typically either on an externally-applied reference patch attached to the exterior of the body of the subject, or on an internally-placed catheter, is maintained in a generally fixed position relative to heart 24. By comparing the position of catheter 30 to that of the reference catheter, the coordinates of catheter 30 are accurately determined relative to the heart, irrespective of motion of the subject. In an exemplary embodiment, ECG 34 and an additional respiration sensor are used to provide heartbeat and respiration motion compensation discussed further below.

Location system control unit 36 receives signals from position sensor 40 (or from external radiators 28 when position sensor 40 generates the energy fields), calculates the location of sensor 40 and catheter 30, and transmits to computer 50 the location information and energy dose information (received from ablation power generator 38, as described below) which relates to the location information. The location system control unit preferably generates and transmits location information essentially continuously.

Ablation power generator 38 preferably generates power used by tip electrode 48 to perform ablation. Preferably, the ablation power generator generates RF power for performing RF ablation. Alternatively or additionally, the ablation power generator induces ablation by means of other ablation techniques, such as laser ablation or ultrasound ablation, for example. Preferably, suitable feedback techniques are applied to facilitate identifying less than suitable ablated regions on the cardiac map, as discussed more fully below.

Ablation power generator 38 measures the current needed to maintain the tip at a constant temperature of between about 50 °C to about 65 °C. The ablation power generator 38 transmits electrical current information related to the current needed to maintain a constant tip temperature and preferably over a serial communications line, to computer 50. The technical means of transportation over a "serial communications line" are not relevant. What is important is that the signal feed is synchronous and real-time capable such that ECG(t), depth of respiration(t), ablation feedback(t) and position(t) are all available close to the time (t) when they have been acquired, which is mentioned later as "essentially continuously". The ablation power generator preferably measures and transmits the electrical current needed to sustain the tip at a constant temperature essentially continuously.

Alternatively, a cardiac map generated during a previous cardiac procedure is used. In an exemplary embodiment, a cardiac map adapted to the patient heart's anatomy is acquired from another source, such as an imaging modality (e.g., fluoroscopy, MRI, echocardiography, CT, single-photon computed tomography (SPECT), or positron emission tomography (PET)), and the location of the catheter is visualized on this map for at least sites of ablation that are not successful because of lack of contact between the tip and tissue of the heart. In this case, computer 50 marks the intended ablation lesion locations on this map as gaps in a line or ring ablation. Alternatively, for some applications, a cardiac map adapted to the anatomy of the patient's heart is not acquired, in which case only a map indicative of a proximate location of where the catheter ablation tip was located is acquired when lack of contact between the tip and tissue is detected.

Figure 4 is a flow chart 200 that schematically illustrates a method for indicating a gap in a line or ring ablation, and thus an incomplete ablation formed in a cardiac chamber, in accordance with an exemplary embodiment of the present disclosure. After a geometric and electrical map of the cardiac chamber has been generated, user 22 advances catheter 30 to the area of the surface of the cardiac chamber on which ablation is to be performed at block 202. As ablation energy is applied to the cardiac surface, ablation power generator 38 measures, preferably continuously, the amount of electrical current that is needed to maintain the tip at a constant temperature at block 204, as described above. Current ablation catheters are controlled to keep a constant temperature at their tip of about 50 °C to about 65°C. The ablation power generator 38 senses the amount of current that is required to heat the catheter tip. A simple threshold decision or other means of signal classification applied to this feedback information at block 206 then allows distinguishing between contact of the catheter tip with cardiac tissue (e.g., low current required) and the catheter tip that is only in contact with the blood flow (e.g., high current required). If the measured feedback signal is classified as "not in contact" with the heart tissue, then block 202. If the measured current is greater than the threshold current value indicative of a lack of tip contact with the heart tissue, then block 208. A system according to the intervention automatically detects lost contact between the tip and cardiac tissue based on this control parameter (e.g., sensed electrical current). The system includes acquiring and storing any relevant available data available defining the current catheter position at block 208. At block 210, the user 22 or interventionalist revisits any gaps in the ablation path using the data acquired at block 208 defining the current position of the catheter tip when the measured current is above the threshold current value indicative of lost contact between the tip and heart tissue.

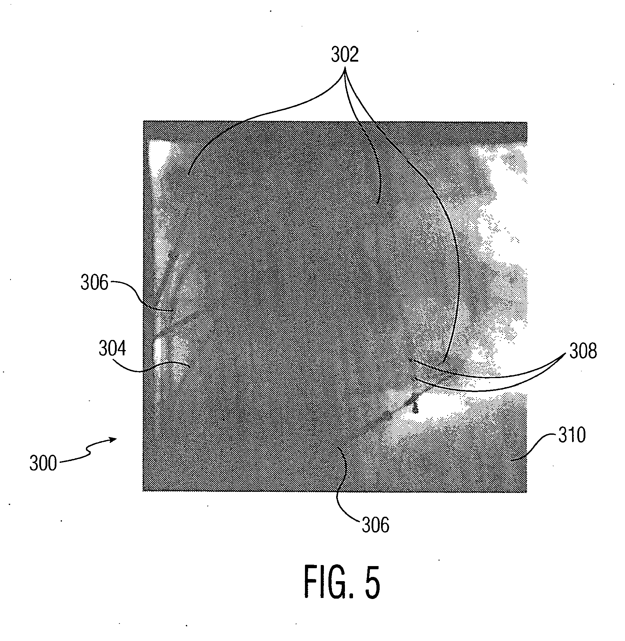

In exemplary embodiments, the data includes current localizer information and x-ray images (Figure 5) of the catheter acquired and stored at the moment that lost contact is detected (e.g., current above a threshold value). Acquisition of these images at the moment of lost contact will indicate the catheter tip proximate to a position where the ablation must be continued or completed to avoid gaps in a line or ring ablation. Figure 5 is a fluoroscopic image 300 acquired when the measured current required to maintain a constant tip temperature rises above the threshold current value. Image 300 illustrates three ECG leads 302 proximate heart 24 attached to the patient's skin. The reference EP catheter 304 in one of the atria is the middle one of the three visible catheters 306, i.e. the dark bend structures. This catheter 304 is positioned at an anatomical landmark, e.g. the coronary sinus. The lower EP catheter 306 appears to lay in the left ventricle close to the apex. On this catheter, radio-opaque marker rings 308 are easily visible. The upper catheter 306 is located in one of the atrial chambers of the heart. The diaphragm 310 separating the lung from abdominal organs is visible in the lower right of the image 300 and is a possible source to determine the depth of respiration intake. Image 300 illustrates the arc-shaped transition from bright lung tissue to darker abdominal tissue. Furthermore, image 300 depicts the spine and some ribs, but these are not of interest.

Two modes of operation are supported using data defining a current position of the catheter tip corresponding with a moment that lost contact between the cardiac tissue and tip is detected. The modes of operation relate to the point in time when the interventionalist makes use of this information, either as soon as the gap candidate has been identified or the ablation is continued as normal and the interventionalist navigates back if and only if the ablation was not successful. By use of mask overlays, i.e. a mixing of live images and images acquired when contact was lost, the current position of the catheter and the position where contact was lost can be presented such that a revisit of the lost position is guided by an image or by localizer geometry and, therefore, easily achievable. In a second mode, a list of candidate positions can be displayed when a finished line or ring ablation has not been successful, which is easily detectable on the ECG 34 as soon as the ablation is considered finished. In this manner, corrections need only be applied to these candidate positions and it is not necessary to retrace the complete ablation procedure.

One proposed embodiment of the invention consists of a software module that is integrated into an EP workstation or console 20 depicted generally at 100 within computer 50. Such an EP workstation is the central control and display unit of an EP procedure and combines the EP-specific ECG signals, x-ray and localizer information. The software module 100 receives data corresponding to the sensed electrical current that is required to heat the ablation catheter tip to the target temperature. When the electrical current rises above a threshold, lost contact between the tip and heart tissue is detected. The software module 100 then instructs computer 50 to automatically store any and all available data that defines the current catheter position together with available information on the patient's status, namely the current cardiac phase determined from ECG and the depth of respiration intake determined from an external sensor or the optionally acquired image.

For example, when localizers are used, then the available data defining current position of the catheter tip includes storing localizer geometry. For ablations under x-ray surveillance, a current fluoroscopy image is stored (FIG. 5). In an extended embodiment of this intervention, the acquisition of one fluoroscopy frame can be automatically triggered (e.g., imager switched on for one frame), even if the fluoroscopy is currently in an off state. The current phase in the heart and respiration motion cycle is acquired using ECG 34 and one of the described means to determine depth of respiration and stored as well to allow for respective motion compensations that are required to position the catheter at the same position with respect to the cardiac tissue independent of motion due to respiration and heart beat.

This information indicative of position of the catheter tip when contact is lost with the heart tissue can be used either in an immediate mode in a revisit mode discussed above. To start the ablation immediately after the contact was lost as close as possible to the previous position, a respiration and heart motion compensated mask overlay of the automatically stored image or the position of the localizers and their distance to the target position are displayed to the interventionalist 22 on monitor 52 such that the user 22 can easily reposition the catheter to continue with the interrupted ablation.

The revisit mode is used when the EP ablation procedure is finished, but the reentry tachycardia is not blocked. It will be recognized that immediate treatment results are obtained once the ablation is complete using ECG 34. Then, the interventionalist 22 is provided with a list of candidate positions where insufficient contact between the catheter and heart tissue was present during ablation and can successively use the navigation support provided in the immediate mode via monitor 52, as described above, for these candidate positions until success of the intervention is obtained.

The offer of advanced dedicated EP lab equipment incorporating the software module 100 as described above offers the assignee of the current application a tremendous opportunity in this growing market. The automated acquisition and storage of position information at the moment when ablation contact to the heart tissue is lost serves as a unique selling proposition for such an EP lab. One advantage includes the massive reduction in the amount of time that is spent in trial and error corrections of incomplete ring and line ablations to treat reentry tachycardia.

All dedicated EP labs may incorporate the EP workstation according to the exemplary embodiments described herein, e.g. a target hardware that controls and combines the various hardware (e.g., x-ray imager, EP ECG acquisition, ablation catheter control, and localizer system). The invention is easily included in a software package for such a workstation.

In sum, the disclosed apparatus, and computer software product provide significant benefits to users of EP workstations, particularly physicians desiring a reduction in the amount of time to complete line and ring ablations to treat reentry tachycardia. The handling of possible gaps in an incomplete line or ring ablation includes automated creation and display of an image of the current catheter tip position or the storage of the current position image for later re-visit, which is necessary if the treatment goal is not reached at the end of the ablation path. Further, the immediate or later re-visit of a gap candidate in the path is simplified when heartbeat and/or respiration motion compensation is provided using an ECG and information on depth of respiration. In this manner, the re-visit can be image-guided using interventional imaging devices or based on localizer information. In contrast to the current use of the localizer information in daily clinical routine and the above described exemplary embodiments, it is proposed to use the localizer information for targeted navigation support. For example, the indication of current position information is replaced with information indicative of where the catheter should be disposed. For example, the localizer information would give the distance and direction to gap candidates from the current position of the tip, rather than information pertaining to only the current position of the ablation tip, and in so doing, applying heartbeat and respiraton motion compensation. For this, the comparison between the position of the gap candidate and the current position of the catheter is corrected for heart and respiration motion using the synchronously acquired ECG and depth of respiration together with information how the catheter moves locally due to heart beat and respiration. The latter information can be extracted by observing the position of the catheter tip over a heart cycle and an independent observation of the motion of the heart in the rib cage due to respiration.

Advantageously, embodiments of the present disclosure enable a user of the apparatus and computer software product to visually determine, in real-time during a procedure, which areas of the surface of the cardiac chamber have not been ablated and which require application or re-application of the ablating electrode. As a result, a more complete non-conducting lesion is typically formed, without unnecessary ablation of excess cardiac tissue and in less time than before possible.

Although the apparatus and software product of the present disclosure have been described with reference to exemplary embodiments thereof, the present disclosure is not limited to such exemplary embodiments. Rather, the apparatus and software product disclosed herein are susceptible to a variety of modifications, enhancements and/or variations, without departing from the spirit or scope hereof. Accordingly, the present disclosure embodies and encompasses such modifications, enhancements and/or variations within the scope of the claims appended hereto.

## Claims

1. An apparatus for ablating tissue in a heart (24) of a subject (25) during an ablation procedure, the apparatus comprising:
an ablation catheter tip (48) for contacting tissue of the heart (24) at a plurality of sites designated for ablation,
a storage means for storing any available data defining a current position of the ablation catheter tip (48) relative to the heart (24), and
a display means (60) for displaying a map of a region of interest of the heart (24),
**characterized by** a sensor means for sensing at each respective site electrical current required to maintain the tip (48) at a target temperature, wherein the storage means is configured for storing data at a moment of sensing the required electrical current above a threshold current value for later re-visit, and wherein the display means is configured for designating indications of the sites corresponding to when the required electrical current is above the threshold current value indicative of a gap in an ablation line or ring.

2. The apparatus according to claim 1, comprising a location system (11) provided with external radiators (28) for generating fields, and a position sensor (40) for detecting said fields.

3. The apparatus according to claim 1, comprising a position sensor (40) for generating fields, and a location system (11) provided with external radiators (28) for detecting said fields.

4. The apparatus according to claim 2 or 3 comprising a reference position sensor in fixed position relative to the heart (24).

5. The apparatus according to claim 4, wherein the reference position sensor is installed at a reference path that is externally applicable to the body of the subject.

6. The apparatus according to claim 4, wherein the reference position sensor is installed at a reference catheter.

7. The apparatus according to claim 1, comprising a respiration sensor for compensating heartbeat and respiration motion.

## Patentansprüche

1. Vorrichtung zum Abladieren von Gewebe im Herzen (24) eines Objekts (25) während einer Ablationsprozedur, wobei die Vorrichtung Folgendes umfasst:
eine Ablationskatheterspitze (48) zum Herstellen des Kontakts mit Gewebe des Herzens (24) an einer Vielzahl von zur Ablation bestimmten Stellen,
Speichermittel zum Speichern aller verfügbaren Daten, die die aktuelle Position der Ablationskatheterspitze (48) bezüglich des Herzens (24) definieren, und
Anzeigemittel (60) zum Anzeigen einer Karte der interessierenden Region des Herzens (24),
**dadurch gekennzeichnet, dass** sie Sensormittel zum Abtasten an jeder entsprechenden Stelle des elektrischen Stroms umfasst, der erforderlich ist, um die Spitze (48) auf der Zieltemperatur zu halten, wobei die Speichermittel so konfiguriert sind, dass sie zur späteren erneuten Kontrolle Daten zum Zeitpunkt der Abtastung des erforderlichen elektrischen Stroms speichern, der oberhalb eines Schwellenstromwertes liegt, und wobei die Anzeigemittel so konfiguriert sind, dass sie Hinweise auf Stellen, die Zeitpunkten entsprechen, zu denen der erforderliche elektrische Strom über dem Schwellenstromwert liegt, als Indiz für eine Lücke in einer Ablationslinie oder einem Ablationsring kennzeichnen.

2. Vorrichtung nach Anspruch 1, die ein Lokalisationssystem (11) umfasst, das mit externen Strahlern (28) zum Erzeugen von Feldern und einem Positionssensor (40) zum Detektieren der genannten Felder ausgestattet ist.

3. Vorrichtung nach Anspruch 1, die einen Positionssensor (40) zum Erzeugen von Feldern und ein Lokalisationssystem (11) umfasst, das mit externen Strahlern (28) zum Detektieren der genannten Felder ausgestattet ist.

4. Vorrichtung nach Anspruch 2 oder 3, die einen Referenzpositionssensor an einer festen Position bezüglich des Herzens (24) umfasst.

5. Vorrichtung nach Anspruch 4, wobei der Referenzpositionssensor in einem Referenzpfad installiert ist, der extern am Körper des Objekts angebracht werden kann.

6. Vorrichtung nach Anspruch 4, wobei der Referenzpositionssensor an einem Referenzkatheter installiert ist.

7. Vorrichtung nach Anspruch 1, die einen Atmungssensor zum Ausgleichen der Bewegung durch den Herzschlag und die Atmung umfasst.

## Revendications

1. Appareil pour réaliser une ablation d'un tissu dans un coeur (24) d'un patient (25) au cours d'une procédure d'ablation, l'appareil comprenant :
un embout de cathéter d'ablation (48) pour entrer en contact avec le tissu du coeur (24) à une pluralité de sites désignés pour l'ablation,
un moyen de stockage pour stocker de quelconques données disponibles définissant une position actuelle de l'embout de cathéter d'ablation (48) par rapport au coeur (24), et
un moyen d'affichage (60) pour afficher une carte d'une région d'intérêt du coeur (24),
**caractérisé par** un moyen capteur pour détecter à chaque site respectif un courant électrique nécessaire pour maintenir l'embout (48) à une température cible, dans lequel le moyen de stockage est configuré pour stocker des données à un instant de détection du courant électrique nécessaire supérieur à une valeur de courant de seuil pour une revisite ultérieure, et dans lequel le moyen d'affichage est configuré pour désigner des indications des sites correspondant à l'instant auquel le courant électrique nécessaire est supérieur à la valeur de courant de seuil indicative d'un espace dans une ligne ou un cercle d'ablation.

2. Appareil selon la revendication 1, comprenant un système de localisation (11) pourvu d'éléments rayonnants externes (28) pour générer des champs, et un capteur de position (40) pour détecter lesdits champs.

3. Appareil selon la revendication 1, comprenant un capteur de position (40) pour générer des champs, et un système de localisation (11) pourvu d'éléments rayonnants externes (28) pour détecter lesdits champs.

4. Appareil selon la revendication 2 ou 3, comprenant un capteur de position de référence dans une position fixe par rapport au coeur (24).

5. Appareil selon la revendication 4, dans lequel le capteur de position de référence est installé dans un trajet de référence qui est applicable extérieurement sur le corps du patient.

6. Appareil selon la revendication 4, dans lequel le capteur de position de référence est installé dans un cathéter de référence.

7. Appareil selon la revendication 1, comprenant un capteur de respiration pour compenser un battement de coeur et un mouvement de respiration.
